# EUROPEAN PATENT SPECIFICATION

(11) **EP 0 664 832 B1**
(45) Date of publication and mention of the grant of the patent: **24.07.2002**
(21) Application number: 93922482.0
(22) Date of filing: 15.10.1993
(51) Int. Cl.: C12N 5/08, C12N 5/00, A61K 35/30

(54) **REMYELINATION USING NEURAL STEM CELLS**
REMYELINATION UNTER VERWENDUNG VON NEURONALEN STAMMZELLEN
REMYELINATION EFFECTUE A L'AIDE DE CELLULES SOUCHES NEURALES

(30) Priority: 16.10.1992 US 961813
(43) Date of publication of application: 02.08.1995
(73) Proprietor: NEUROSPHERES HOLDINGS LTD., Calgary, Alberta T2N 4N1 (CA)
(72) Inventor: WEISS, Samuel, Calgary, Alberta T2L 1A6 (CA); REYNOLDS, Brent, A., Calgary, Alberta T3B 2V6 (CA); HAMMANG, Joseph, P., Barrington, RI 02806 (US)
(74) Representative: Baldock, Sharon Claire
(86) International application number: CA9300428
(87) International publication number: WO9409119

(56) References cited:
- WO-A-91/02003
- WO-A-91/09936
- WO-A-93/01275
- SCIENCE vol. 255 , 27 March 1992 , WASHINGTON D.C.,USA pages 1707 - 1710 REYNOLDS ET AL 'GENERATION OF NEURONS AND ASTROCYTES FROM ISOLATED CELLS OF THE ADULT MAMMALIAN CENTRAL NERVOUS SYSTEM' cited in the application
- FILE SERVER STN KARLSRUHE,FILE BIOSIS ABSTRACT NO.91:324328 & RADIOTHER ONCOL 20 (4) 1991 258-264
- THE JOURNAL OF CELL BIOLOGY vol. 109, no. 5 , November 1989 , NEW YORK,USA pages 2405 - 2416 GODFRAIND ET AL 'IN VIVO ANALYSIS OF GLIAL CELL PHENOTYPES DURING A VIRAL DEMYELINATING DISEASE IN MICE' cited in the application

## Description

### FIELD OF THE INVENTION

The present invention is directed to the uses of neural stem cells (NSCs) and their descendants to remyelinate axons. More particularly, the invention is related to the treatment of demyelinating diseases by the remyelination of neurons through the addition of exogenous myelin forming cells and precursors thereof.

### BACKGROUND OF THE INVENTION

Myelin is a cellular sheath, formed by glial cells, that surrounds axons and axonal processes that enhances various electrochemical properties and provides trophic support to the neuron. Myelin is formed by Schwann cells in the peripheral nervous system and by oligodendrocytes in the central nervous system.

Demyelination of central and peripheral neurons occurs in a number of pathologies and leads to improper signal conduction within the nervous systems. Among the various demyelinating diseases Multiple Sclerosis (MS) is the most notable.

In both human demyelinating diseases and rodent models there is substantial evidence that demyelinated neurons are capable of remyelination in situ. In MS, for example, it appears that there are often cycles of de- and remyelination. Similar observations in rodent demyelinating paradigms lead to the prediction that exogenously applied cells would be capable of remyelinating demyelinated axons. This approach has proven successful in a number of experimental conditions [Freidman et al., Brain Research, 378:142-146 (1986); Raine, et al., Laboratory Investigation 59:467-476 (1988); Duncan et al., J. of Neurocytology, 17:351-360 (1988)]. The sources of cells for some of these experiments included dissociated glial cell suspensions prepared from spinal cords (Duncan et al., supra), Schwann cell cultures prepared from sciatic nerve [Bunge et al., 1992, WO 92/03536; Blakemore and Crang, J. Neurol. Sci., 70:207-223 (1985)]; cultures from dissociated brain tissue [Blakemore and Crang, Dev. Neurosci. 10:1-11 (1988)], oligodendrocyte precursor cells [Gumpel et al., Dev. Neurosci. 11:132-139 (1989)], O-2A cells [Wolswijk et al., Development 109:691-608 (1990); Raff et al., Nature 3030:390-396 (1983); Hardy et al., Development 111:1061-1080 (1991)], and immortalized O-2A cell lines, [Almazan and McKay Brain Res. 579:234-245 (1992)].

O-2A cells are glial progenitor cells which give rise in vitro only to oligodendrocytes and type II astrocytes. Cells which appear by immunostaining in vivo to have the O-2A phenotype have been shown to successfully remyelinate demyelinated neurons in vivo. Godfraind et al., J. Cell Biol. 109:2405-2416 (1989). Injection of a large number of O-2A cells is required to adequately remyelinate all targeted neurons in vivo, since it appears that O-2A cells (like other glial cell preparations) do not continue to divide in situ. Although O-2A progenitor cells can be grown in culture, currently the only available isolation technique employs optic nerve as starting material. This is a low yield source, which requires a number of purification steps. There is an additional drawback that O-2A cells isolated by the available procedures are capable of only a limited number of divisions. Raff Science 243:1450-1455 (1989).

For in vivo remyelination, it would be advantageous to inject only a small number of cells which could go on to divide, migrate to appropriate targets, and differentiate in situ. It has been shown that the presence of type I astrocytes is important for remyelination to occur with exogenously added oligodendrocytes. The use of oligodendrocyte precursors such as O-2A cells requires that type I astrocytes be co-injected, or alternatively that platelet-derived growth factor (PDGF) be provided to the remyelinating cells. PDGF is a potent mitogen for the O-2A precursor and is secreted from type 1 astrocytes.

Crude cell preparations and suspensions are not preferred as exogenous sources of remyelinating cells because they contain an unpredictable number of cells as well as large numbers of both neural and non-neural cell types, thus making reproducibility of the process difficult. It is also difficult to obtain suitable numbers of cells from these preparations.

Transformed O-2A cell lines are unsuitable for transplantation due to the fact that the transformation process leads to a genetically (oncogene) controlled cell division as opposed to primary cell lines or neural stem or progenitor cells where regulation of division is at an epigenetic level. Additional potential problems include instability of cell lines over long periods of time, and aberrant patterns of differentiation or responses to growth factors. Goldman Trends Neuro. Sci. 15:359-362 (1992).

Thus there exists a need for a reliable source of cells for remyelination therapy. Preferably cellular division in such cells from such a source would be epigenetically regulated and a suitable number of cells could be efficiently prepared in sufficient numbers to effect remyelination. The cells should be suitable in autografts, xenografts, and allografts without a concern for tumor formation.

Accordingly, it is an object of this invention to provide a reliable source of epigenetically regulated cells for transplantation, which are capable of differentiating into oligodendrocytes.

It is another object of this invention to provide a treatment for myelin deficient recipients, which comprises precursor cells derived from stem cells proliferated in vitro, which cells are transplanted into a myelin deficient recipient wherein the cells differentiate into oligodendrocytes thus effecting remyelination of the recipient's axons.

These and other objects and features of the invention will be apparent to those skilled in the art from the following detailed description and appended claims when taken in conjunction with the figures.

None of the foregoing references is believed to disclose the present invention as claimed and is not presumed to be prior art. The references are offered for the purpose of background information.

A method of remyelinating neurons is described as well as cell cultures for use in such a method. In accordance with the invention isolated neural stem cells are proliferated, in vitro, in culture medium containing a growth factor which induces the production of precursor cells. The precursor cells are harvested for use under appropriate conditions to effect the remyelination of demyelinated axons. Alternatively, the precursor cells are allowed to differentiate into oligodendrocytes in the presence of a culture medium which is substantially free of the stem cell-proliferating growth factor. The precursor cell-derived oligodendrocytes are also for associating with demyelinated axons to effect remyelination.

### DETAILED DESCRIPTION OF THE INVENTION

### Definitions

The term "stem cell" refers to an undifferentiated cell which is capable of proliferation and giving rise to more stem cells having the ability to generate a large number of progenitor cells that can in turn give rise to differentiated, or differentiable daughter cells.

The term "neural stem cell" (NSC) refers to the stem cells of the instant invention, the progeny of which under appropriate culturing conditions, include both glial and neuronal progenitor cells.

The term "progenitor cells" refers to the undifferentiated cells of the instant invention, derived from neural stem cells, the progeny of which may, under appropriate conditions, include glial and/or neuronal progenitor cells.

The term "oligodendrocyte" refers to a differentiated glial cell which forms the myelin surrounding axons in the central nervous system (CNS). Oligodendrocytes are of the phenotype galactocerebroside (+), myelin basic protein (+), and glial fibrillary acidic protein (-) [Gal C(+), MBP(+), GFAP(-)].

The term "type I astrocyte" refers to a differentiated glial cell type with a flat protoplasmic/fibroblast-like morphology that is GFAP(+), Gal C(-), and MBP(-).

The term "type II astrocyte" refers to a differentiated glial cell displaying a stellate process bearing morphology of the phenotype GFAP(+), Gal C(-), and MBP(-).

The term "neuronal progenitor cells" refers to cells which produce daughter cells which under the appropriate conditions become or give rise to neurons.

The term "oligodendrocyte precursor cells" refers to cells which give rise to oligodendrocytes. Oligodendrocyte precursor cells can have the phenotype A2B5(+), O4(+)/Gal C(-), MBP(-) and GFAP (-) [but are not limited to this phenotype].

The term "neurosphere" refers to a cluster of cells derived from neural stem cells and cultured in vitro. At least some of the cells are of the nestin (+) phenotype. The cluster is comprised of stem cells and/or progenitor cells and may or may not include differentiated cells.

The term "precursor cells" refers to the living cells of the instant invention that are derived from neural stem cells proliferated in a culture medium containing a growth factor, and includes both progenitor and stem cells. Precursor cells typically grow in the form of neurospheres, but may exhibit different growth patterns depending upon culture conditions.

The term "growth factor" refers to a protein or peptide having a growth or trophic effect.

The term "donor" refers to the human or animal which is the source of the neural stem cells used in the instant invention.

The term "harvesting" refers to any method used to procure proliferated cells in a form suitable for injection or transplantation.

The term "recipient" refers to the human or animal that has demyelinated axons and into which the precursor cells or oligodendrocytes derived from the precursor cells are transplanted or injected.

### Brief Description of the Drawings

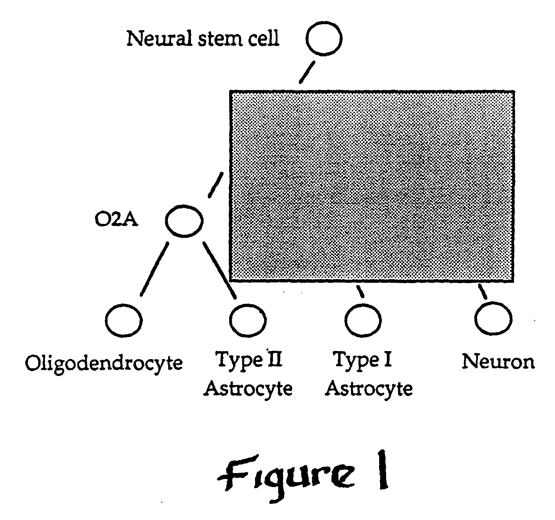

Figure 1 indicates only a single progenitor cell for oligodendrocytes and type II astrocytes. In fact, various reports indicate there may be multiple progenitor cells for these differentiated phenotypes. Goldman, TINS 15:359-362 (1992). An important aspect of the diagram is that neural stem cells are unique in that they are the only stem cell capable of giving rise to both neurons and glial in vitro.

### Description of the Preferred Embodiments

### Phenotypical characteristics

Neural stem cells (NSCs) have been reported and their potential use described. (Reynolds and Weiss, Science 255:1707 (1992)). NSCs have been shown to give rise to at least three glial phenotypes including oligodendrocytes and type I and II astrocytes. NSCs also give rise to neuroblasts. (Reynolds and Weiss, Restorative Neurology & Neuroscience 4:208 (1992)).

Neural stem cells can be isolated and cultured by the method of Reynolds and Weiss (supra). In brief, the epidermal growth factor (EGF) responsive stem cell, when grown in a defined serum-free medium, and in the presence of a mitogen such as EGF or the like, is induced to divide giving rise to a cluster of undifferentiated cells. The cluster of cells are not immunoreactive for GFAP, neural filament (NF), neuron specific enolase (NSE) or MBP. However, precursor cells within the cluster are immunoreactive for nestin, an intermediate filament protein found in undifferentiated CNS cells. The nestin marker was characterized by Lehndahl et al., Cell 60:585-595 (1990), and is incorporated herein by reference. None of the mature phenotypes associated with the four cell types which may be differentiated from the progeny of the precursor cells have the nestin phenotype.

In the continued presence of a mitogen such as EGF or the like, precursor cells within the neurosphere continue to divide resulting in an increase in the size of the neurosphere and the number of undifferentiated cells [nestin(+), GFAP(-), NF(-), NSE (-), MBP (-)]. At this stage the cells are non-adherent and tend to form the free-floating clusters characteristic of neurospheres. However, culture conditions may be varied so that while the precursor cells still express the nestin phenotype, they do not form the characteristic neurospheres. After removal of the mitogen the cells adhere to the substrate (poly-ornithine-treated plastic or glass), flatten, and begin to differentiate into neurons and glial cells. At this stage the culture medium may contain serum such as 0.5-1.0% fetal bovine serum (FBS). Within 2-3 days, most or all of the precursor cells begin to lose immunoreactivity for nestin and begin to express intermediate filaments specific for neurons or for astrocytes as indicated by immunoreactivity to NFL or GFAP respectively. In addition, a large number of cells that do not express either of these intermediate filament markers, begin to express markers specific for oligodendrocytes in a correct temporal fashion. That is, the cells first become immunoreactive for O4 (a cell surface antigen), galactocerebroside (Gal C, a myelin glycolipid) and finally, myelin basic protein (MBP). These cells also possess a characteristic oligodendrocyte morphology. This information considered in light of the oligodendrocyte progenitor cells identified by Raff gives rise to a number of possible cell lineage relationships. (See Figure 1).

### Preparation of Neurospheres

Neurospheres can be generated from a variety of tissues including adult or fetal neural tissue from human or animal sources. Briefly, individual stem cells are prepared from the dissociation of neural tissues. Dissociation can be obtained using any known procedure, including treatment with enzymes such as trypsin, collagenase and the like,or by using physical methods of dissociation such as with a blunt instrument. Dissociation of fetal cells can be carried out in tissue culture medium, while a preferable medium for dissociation of adult cells is artificial cerebral spinal fluid (aCSF). Regular aCSF contains 124 mM NaCl, 5 mM KCl, 1.3 mM MgCl₂, 2 mM CaCl₂, 26 mM NaHCO₃, and 10 mM D-glucose. Low Ca⁺⁺ aCSF contains the same ingredients except for MgCl₂ at a concentration of 3.2 mM and CaCl₂ at a concentration of 0.1 mM.

The individual cells can be placed into any known culture medium capable of supporting cell growth and proliferation, including MEM, DMEM, RPMI, F-12, and the like, containing supplements which are required for cellular metabolism such as glutamine and other amino acids, vitamins, minerals and useful proteins such as transferrin and the like. Medium may also contain antibiotics to prevent contamination with yeast, bacteria and fungi such as penicillin, streptomycin, gentamicin and the like. The dissociated cells form neurospheres in the presence of a mitogen such as EGF or the like.

### Differentiation of Glial Cells from Neurospheres

Astrocytes and oligodendrocytes can be differentiated from the precursor cells of the neurosphere by placing the neurospheres in a medium containing 1% fetal bovine serum in the absence of a mitogen, such as EGF or the like, for approximately 3-4 days on poly-ornithine treated glass or plastic. These cells can then be maintained in culture for a suitable time period to produce large numbers of differentiated cells. Cultures can then be purified to a single cell type using any of the methods known to the art for the purification of specific glial cell populations. Some preferred approaches in this regard are the immunological methods of Wolswijk et al., Development 109:691-698 (1990) and those of Franklin et al., J. Neurocytology 20:420-430 (1991).

### Use of Differentiated Cells

Differentiated cells in the form of oligodendrocytes that are derived from precursor cells may be injected into demyelinated target areas in the recipient. Appropriate amounts of type I astrocytes may also be injected. Type I astrocytes are known to secrete PDGF which promotes both migration and cell division of oligodendrocytes. [Nobel et al., Nature 333:560-652 (1988); Richardson et al., Cell, 53:309-319 (1988)].

### Use of Non-differentiated Precursor Cells

Non-differentiated precursor cells are preferred as the cells for treatment of demyelinating diseases. Neurospheres grown in the presence of EGF can be dissociated to obtain individual precursor cells which are then placed in injection medium and injected directly into the demyelinated target region. Astrocytes can promote remyelination in various paradigms. Therefore, in instances where oligodendrocyte proliferation is important, the ability of precursor cells to give rise to type I astrocytes may be useful. In other situations, PDGF may be applied topically during the transplantation as well as with repeated doses to the implant site thereafter.

The injection of precursor cells in remyelination therapy provides a source of immature type I astrocytes at the implant site. This is a significant feature because immature astrocytes (as opposed to mature astrocytes) have a number of specific characteristics that make them particularly suited for remyelination therapy. First, immature, as opposed to mature, type I astrocytes are known to migrate away from the implant site [Lindsay et. al, Neurosci. 12:513-530 (1984)] when implanted into a mature recipient and become associated with blood vessels in the recipient's CNS [Silver et al., WO 91/06631 (1991)]. This is at least partially due to the fact that immature astrocytes are intrinsically more motile than mature astrocytes. [Duffy et al., Exp Cell Res. 139:145-157 (1982), Table VII]. Type I astrocytes differentiating at or near the precursor cell implant site should have maximal motility and thereby optimize the opportunity for oligodendrocyte growth and division at sites distant from the implant. The localization of the astrocytes near blood vessels is also significant from a therapeutic standpoint since (at least in MS) most plaques have a close anatomical relationship with one or more veins.

Another characteristic of immature astrocytes that makes them particularly suited for remyelination therapy is that they undergo a lesser degree of cell death than mature type I astrocytes. (Silver et al., supra)

### Implantation

Any suitable method for the implantation of precursor cells near to the demyelinated targets may be used so that the cells can become associated with the demyelinated axons. Glial cells are motile and are known to migrate to, along, and across their neuronal targets thereby allowing the spacing of injections. Injection methods exemplified by those used by Duncan et al. J.Neurocytology, 17:351-361 (1988), incorporated herein by reference, and scaled up and modified for use in humans are preferred. Methods taught by Gage et al. US Patent No. 5,082,670, incorporated herein by reference, for the injection of cell suspensions such as fibroblasts into the CNS may also be employed for injection of precursor cells. Additional approaches and methods may be found in Neural Grafting in the Mammalian CNS, Bjorklund and Stenevi, eds., (1985), incorporated herein by reference.

### Autografts

In some instances, it may be possible to prepare precursor cells from the recipient's own nervous system (e.g.in the case of tumor removal biopsies etc,). In such instances the precursor cells may be generated from dissociated tissue and grown in culture in the presence of a mitogen such as EGF or the like, or basic fibroblast growth factor (bFGF). Upon suitable expansion of cell numbers, the precursor cells may be harvested and readied for direct injection into the recipient's CNS. In the case of demyelinating diseases with a genetic basis directly affecting the ability of the myelin forming cell to myelinate axons, it will generally not be useful to remyelinate using the recipients cells as donor cells, unless the cells have been modified in some way to insure the lesion will not continue (e.g. genetically modifying the cells to cure the demyelination lesion).

Xeno and/or allografts may require the application of immunosuppressive techniques or induction of host tolerance to insure longevity of remyelination. Local immunosuppression is disclosed by Gruber, Transplantation 54:1-11 (1992), incorporated by reference. Rossini, US Patent No. 5,026,365, discloses encapsulation methods suitable for local immunosuppression. General reviews and citations for the use of recombinant methods to reduce antigenicity of donor cells are disclosed by Gruber (supra). Exemplary approaches to the reduction of immunogenicity of transplants by surface modification are disclosed by Faustman WO 92/04033 (1992).

### Xenografts

The instant invention allows the use of precursor cells prepared from donor tissue which is xenogeneic to the host. Since the CNS is a somewhat immunoprivileged site, the immune response is significantly less to xenografts, than elsewhere in the body. In general, however, in order for xenografts to be successful it is preferred that some method of reducing or eliminating the immune response to the implanted tissue be employed. Thus recipients will often be immunosuppressed, either through the use of immunosuppressive drugs such as cyclosporin, or through local immunosuppression strategies employing locally applied immunosuppressants. Alternatively the immunogenicity of the graft may be reduced by preparing precursor cells from a donor with reduced antigenicity, such as transgenic animals which have altered or deleted MHC antigens.

### Allografts

Grafting of precursor cells prepared from tissue which is allogeneic to that of the recipient will most often employ tissue typing in an effort to most closely match the histocompatibility type of the recipient. Donor cell age as well as age of the recipient have been demonstrated to be important factors in improving the probability of neuronal graft survival. The efficiency of grafting is reduced with increased age of donor cells. Furthermore, grafts are more readily accepted by younger recipients compared to older recipients. These two factors are likely to be as important for glial graft survival as they are for neuronal graft survival.

### Implantation in Humans

Areas of demyelination in humans is generally associated with plaque like structures. Plaques can be visualized by magnetic resonance imaging. Accessible plaques are the target area for injection of NSCs. Standard stereotactic neurosurgical methods are used to inject cell suspensions both into the brain and spinal cord.

Remyelination by the injection of precursor cells is a useful therapeutic in a wide range of demyelinating conditions. It should also be borne in mind that in some circumstances remyelination by precursor cells will not result in permanent remyelination, and repeated injections will be required. Such therapeutic approaches offer advantage over leaving the condition untreated and may spare the recipient's life.

A list of human demyelinating diseases for which the cells of the present invention may provide treatment is as follows: disseminated perivenous encephalomyelitis, multiple sclerosis (Charcot and Marburg types), neuromyelitis optica, concentric sclerosis, acute, disseminated encephalomyelitides, post encephalomyelitis, postvaccinal encephalomyelitis, acute hemorrhagic leukoencephalopathy, progressive multifocal leukoencephalopathy, idiopathic polyneuritis, diphtheric neuropathy, Pelizaeus-Merzbacher disease, neuromyelitis optica, diffuse cerebral sclerosis, central pontine myelinosis, spongiform leukodystrophy leukodystrophy (Alexander type).

### Examples

### Example 1

### Propagation of precursor cells for transplantation

Embryonic day 15 (E15) Sprague Dawley rats are decapitated and the brain and striata are removed using sterile procedure. Tissue is mechanically dissociated with a fire-polished Pasteur pipette into serum-free medium composed of a 1:1 mixture of Dulbecco's modified Eagle's medium (DMEM) and F-12 nutrient mixture (Gibco). The cells are centrifuged at 800 r.p.m. for 5 minutes, the supernatant aspirated, and the cells resuspended in DMEM/F-12 medium for counting.

The cells are suspended in a serum-free medium composed of DMEM/F-12 (1:1) including glucose (0.6%), glutamine (2 µM), sodium bicarbonate (3 mM), and HEPES (4-[2-hydroxyethyl]-1-piperazineethanesulfonic acid) buffer (5 mM) (all from Sigma except glutamine [Gibco]). A defined hormone mix and salt mixture (Sigma) that includes insulin (25 µg/ml), transferrin (100 µg/ml), progesterone (20 nM), putrescine (60 µM), and selenium chloride (30 nM) is used in place of serum. In addition, the medium contains 16-20 ng/ml EGF (purified from mouse submaxillary, Collaborative Research) or TGFα (human recombinant, Gibco). The cells are seeded in a T25 culture flask and housed in an incubator at 37°C, 100% humidity, 95% air/5% CO₂. Cells proliferate within 3-4 days and, due to lack of substrate, lift off the floor of the flask and continue to proliferate in suspension forming clusters of undifferentiated precursor cells known as neurospheres.

After 6-8 days in vitro (DIV) the neurospheres are removed, centrifuged at 400 r.p.m. for 2-5 minutes, and the pellet mechanically dissociated into individual cells with a fire-polished glass pasteur pipet. Cells are replated in the growth medium where proliferation of the stem cells and formation of new neurospheres is reinitiated. This procedure is repeated weekly and results in a logarithmic increase in the number of viable cells at each passage. The procedure is continued until the desired number of precursor cells is obtained.

### Example 2

### Remyelination of myelin deficient rats

Litters of first day postnatal myelin deficient rats are anesthetized using ice to produce hypothermia. Myelin deficiency is an X-linked trait and thus only one half of the males in any litter are affected. Therefore, only the males are used for these studies. Once anesthetized, a small rostral to caudal incision is made at the level of the lumbar enlargement. The muscle and connective tissue is removed to expose the vertebral laminae. Using a fine rat tooth forceps, one lamina at the lumbar enlargement is removed and a small cut is made in the dura mater to expose the spinal cord.

A stereotaxic device holding a glass pipet is used to inject a 1 µl aliquot of the cell suspension (approximately 50,000 cells/µl) described above. The suspension is slowly injected into a single site (although more could be done) in the dorsal columns of the spinal cord. As controls, some of the animals are sham-injected with sterile saline. The animals are marked by clipping either toes or ears to distinguish between both experimental groups. Following injection of the cell suspension, the wound is closed using sutures or stainless steel wound clips and the animals are revived by warming on a surgical heating pad and then returned to their mother.

The animals are allowed to survive for three weeks post-injection and are then deeply anesthetized with nembutal (150 mg/kg) and perfused through the left ventricle. The tissues are then dissected from the animal and fixed for 1-3 days with 4% paraformaldehyde in PBS and 95% ethanol/5% acetic acid, respectively and then processed for epoxy embedding. One micron plastic sections are cut with an ultramicrotome and heat-sealed on glass microscope slides and either stained with alkaline toluidine blue (a histological stain for myelin) or processed for immunocytochemistry for the major myelin proteins. Because the myelin deficient rat spinal cord is almost completely devoid of myelin, myelin formed at or near the site of injection will be derived from the implanted cells. It is possible that the process of injection will allow for the entry of Schwann cells (myelinating cells of the peripheral nervous system) into the spinal cord. These cells are capable of forming myelin within the central nervous system but can be easily distinguished from oligodendrocytes using either light microscopy or immunocytochemistry for CNS myelin elements. As noted above, there is usually a very small amount of CNS myelin within the myelin deficient rat spinal cord. This myelin can be distinguished from normal donor myelin based on the mutation within the gene for the major CNS myelin protein, proteolipid protein (PLP). The myelin deficient rat myelin is not immunoreactive for PLP while the donor myelin is.

The myelinated axons are found not only at the site of injection but also in adjacent vertebral sections indicating that the injected precursor cells both migrate away from the site of injection and differentiate to oligodendrocytes in order to form myelin.

### Example 3

### Remyelination in human Neuromyelitis optica

Neuromyelitis optica is a condition involving demyelination of principally the spinal cord and optic nerve. Onset is usually acute and in 50% of the cases death occurs within months. The severity of demyelination as well as lesion sites can be confirmed by magnetic resonance imaging (MRI).

Precursor cells are prepared from fetal human tissue by the method of Example 1. Cells are stereotactically injected into the white matter of the spinal cord in the vicinity of plaques as visualized by MRI. Cells are also injected around the optic nerve as necessary. Booster injections may be performed as required.

### Example 4

### Remyelination in human Pelizaeus-Merzbacher disease

Pelizaeus-Merzbacher disease is a condition involving demyelination of the CNS. The severity of demyelination as well as lesion sites can be confirmed by magnetic resonance imaging (MRI).

Precursor cells are prepared from fetal human tissue by the method of Example 1. Cells are stereotactically injected into the white matter of the spinal cord in the vicinity of plaques as visualized by MRI. Cells are also injected around the optic nerve as necessary. Booster injections may be performed as required.

## Claims

1. Neural stem cells isolated from the tissue of a donor which have been proliferated in culture medium containing a growth factor to produce precursor cells which cells are harvested, for use in effecting remyelination of a demyelinated axon *in vivo.*

2. Neural stem cells for the use as claimed in claim 1 wherein the growth factor is epidermal growth factor.

3. Neural stem cells for the use as claimed in claim 1 wherein said precursor cells are in neurospheres.

4. Neural stem cells for the use as claimed in claim 1 wherein said harvested precursor cells are re-administered to the donor for effecting remyelination of a demyelinated axon.

5. Neural stem cells for the use as claimed in claim 1 wherein said harvested precursor cells are administered to a human.

6. Neural stem cells for the use as claimed in claim 1 or claim 2 wherein said use comprises administration of said harvested precursor cells to a patient having a demyelinating disease.

7. Neural stem cells for the use as claimed in claim 6 wherein said demyelinating disease is selected from the group consisting of multiple sclerosis, disseminated perivenous encephalomyelitis, neuromyelitis optica, concentric sclerosis, acute disseminated encaphalomyelitides, post encephaloymelitis, acute hemorrhagic leukoencephalopathy, progressive multifocal leukoencaphalopathy, idiopathic polyneuritis, diphtheria neuropathy, Pelizaeus-Merzbacher disease, neuromyelitis optica, diffuse cerebal sclerosis, central pontine myelinosis and leukodystrophy.

8. Use of isolated neural stem cells which have been proliferated in culture medium containing a growth factor to produce precursor cells which cells are harvested, in the manufacture of a cell preparation for effecting remyelination of a demyelinated axon *in vivo.*

9. The use as claimed in claim 8 wherein said growth factor is epidermal growth factor.

10. The use as claimed in claim 8 wherein said precursor cells are in neurospheres.

11. The use as claimed in claim 8 wherein said neural stem cells have been isolated from the tissue of a donor and said harvested precursor cells are for administration to said donor.

12. The use as claimed in claim 8 wherein said harvested precursor cells are for administration to a human.

13. The use as claimed in claim 8 or claim 9 wherein said harvested precursor cells are for administration to a patient having a demyelinating disease.

14. The use as claimed in claim 13 wherein said disease is selected from the group consisting of multiple sclerosis, disseminated perivenous encephalomyelitis, neuromyelitis optica, concentric sclerosis, acute disseminated encaphalomyelitides, post encephaloymelitis, acute hemorrhagic leukoencephalopathy, progressive multifocal leukoencaphalopathy, idiopathic polyneuritis, diphtheria neuropathy, Pelizaeus-Merzbacher disease, neuromyelitis optica, diffuse cerebal sclerosis, central pontine myelinosis and leukodystrophy.

15. Oligodendrocytes which have been produced by a method comprising the steps of:
(a) isolating neural stem cells from the tissue of a donor,
(b) proliferating the isolated neural stem ells in a first culture medium containing a growth factor to produce precursor cells,
(c) differentiating the precursor cells in a second culture medium that is substantially free of said growth factor to produce the oligodendrocytes, for use in effecting remyelination of a demyelinated axon *in vivo.*

16. Oligodendrocytes for use as claimed in claim 15 wherein the culture medium of step (c) contains serum.

17. Oligodendrocytes for use as claimed in claim 15 wherein the growth factor is epidermal growth factor.

18. Oligodendrocytes for use as claimed in claim 15 wherein said use comprises administration of said oligodendrocytes to a patient with a demyelinating disease.

19. Oligodendrocytes for use as claimed in claim 18 wherein the disease is selected from the group consisting of multiple sclerosis, disseminated perivenous encephalomyelitis, neuromyelitis optica, concentric sclerosis, acute disseminated encaphalomyelitides, post encephaloymelitis, acute hemorrhagic leukoencephalopathy, progressive multifocal leukoencaphalopathy, idiopathic polyneuritis, diphtheria neuropathy, Pelizaeus-Merzbacher disease, neuromyelitis optica, diffuse cerebal sclerosis, central pontine myelinosis and leukodystrophy.

20. Use of oligodendrocytes which have been produced by a method comprising the steps of:
(a) obtaining neural stem cells,
(b) proliferating the neural stem cells in a first culture medium contain a growth factor to produce precursor cells,
(c) differentiating the precursor cells in a second culture medium that is substantially free of said growth factor to produce ologodendrocytes, in the manufacture of a cell preparation for effecting remyelination of a demyelinated axon *in vivo.*

21. The use as claimed in claim 20 wherein the culture medium of step (c) contains serum.

22. The use as claimed in claim 20 wherein the growth factor is epidermal growth factor.

23. The use as claimed in claim 20 wherein said oligodendrocytes are for administration to a patient having a demyelinating disease.

24. The use as claimed in claim 23 wherein said demyelinating disease is selcted from the group consisting of multiple sclerosis, disseminated perivenous encephalomyelitis, neuromyelitis optica, concentric sclerosis, acute disseminated encaphalomyelitides, post encephaloymelitis, acute hemorrhagic leukoencephalopathy, progressive multifocal leukoencaphalopathy, idiopathic polyneuritis, diphtheria neuropathy, Pelizaeus-Merzbacher disease, neuromyelitis optica, diffuse cerebal sclerosis, central pontine myelinosis and leukodystrophy.

25. A method of producing glial cells comprising the steps of:
(a) taking neural stem cells isolated from a donor,
(b) proliferating the isolated neural stem cells in a first culture medium containing a growth factor to produce precursor cells, and
(c) differentiating the precursor cells in a second culture medium that is substantially free of said growth factor to obtain glial cells.

26. The method of claim 25 wherein the culture medium is step (c) contains serum.

27. The method of claim 25 wherein the glial cells are oligodendrocytes.

28. The method of claim 25 wherein the glial cells are astrocytes.

29. The method of claim 25 wherein the growth factor is epidermal growth factor.

30. The method of claim 25 wherein the precursor cells of step (b) are in neurospheres.

## Patentansprüche

1. Neuronale Stammzellen, isoliert aus dem Gewebe eines Spenders, zur Durchführung einer Remyelinisierung eines demyelinisierten Axons *in vivo,* welche in einem Kulturmedium, das einen Wachstumsfaktor enthält, proliferiert wurden, um Vorläuferzellen zu erzeugen, die geerntet werden.

2. Neuronale Stammzellen für die Verwendung nach Anspruch 1, wobei der Wachstumsfaktor der Epidermis-Wachstumsfaktor ist.

3. Neuronale Stammzellen für die Verwendung nach Anspruch 1, wobei die Vorläuferzellen in Neurokörpern vorliegen.

4. Neuronale Stammzellen für die Verwendung nach Anspruch 1, wobei die geernteten Vorläuferzellen dem Spender wieder verabreicht werden, um die Remyelinisierung eines demyelinisierten Axons durchzuführen.

5. Neuronale Stammzellen für die Verwendung nach Anspruch 1, wobei die geernteten Vorläuferzellen einem Menschen verabreicht werden.

6. Neuronale Stammzellen für die Verwendung nach Anspruch 1 oder Anspruch 2, wobei die Verwendung die Verabreichung der geernteten Vorläuferzellen an einen Patienten mit einer Demyelinisierungserkrankung umfasst.

7. Neuronale Stammzellen für die Verwendung nach Anspruch 6, wobei die Demyelinisierungserkrankung ausgewählt ist aus der Gruppe bestehend aus Multipler Sklerose, disseminierter perivenöser Enzephalomyelitis, optischer Neuromyelitis, konzentrischer Sklerose, akuter disseminierter Enzephalomyelitis (*acute disseminated encaphalomyelitides*), Postenzephalomyelitis, akuter hämorrhagischer Leukoenzephalopathie, progressiver multifokaler Leukoenzephalopathie, Neuronitis, diphtherischer Neuropathie (*diphtheria neuropathy*), Pelizaeus-Merzbacher Erkrankung, optischer Neuromyelitis, diffuser Zerebralsklerose, zentraler pontiner Myelinolyse und Leukodystrophie.

8. Verwendung von isolierten neuronalen Stammzellen zur Herstellung einer Zellzusammensetzung zur Durchführung einer Remyelinisierung eines demyelinisierten Axons *in vivo,* welche in einem Kulturmedium, das einen Wachstumsfaktor enthält, proliferiert wurden, um Vorläuferzellen zu erzeugen, die geerntet werden.

9. Verwendung nach Anspruch 8, wobei der Wachstumsfaktor der Epidermis-Wachstumsfaktor ist.

10. Verwendung nach Anspruch 8, wobei die Vorläuferzellen in Neurokörpern vorliegen.

11. Verwendung nach Anspruch 8, wobei die neuronalen Stammzellen aus dem Gewebe eines Spenders isoliert wurden und die geernteten Vorläuferzellen für die Verabreichung an den Spender bestimmt sind.

12. Verwendung nach Anspruch 8, wobei die geernteten Vorläuferzellen für die Verabreichung an einen Menschen bestimmt sind.

13. Verwendung nach Anspruch 8 oder Anspruch 9, wobei die geernteten Vorläuferzellen für die Verabreichung an einen Patienten mit einer Demyelinisierungserkrankung bestimmt sind.

14. Verwendung nach Anspruch 13, wobei die Erkrankung ausgewählt ist aus der Gruppe bestehend aus Multipler Sklerose, disseminierter perivenöser Enzephalomyelitis, optischer Neuromyelitis, konzentrischer Sklerose, akuter disseminierter Enzephalomyelitis (*acute disseminated encaphalomyelitides*), Postenzephalomyelitis, akuter hämorrhagischer Leukoenzephalopathie, progressiver multifokaler Leukoenzephalopathie, Neuronitis, diphtherischer Neuropathie (*diphtheria neuropathy*), Pelizaeus-Merzbacher Erkrankung, optischer Neuromyelitis, diffuser Zerebralsklerose, zentraler pontiner Myelinolyse und Leukodystrophie.

15. Oligodendrocyten zur Durchführung einer Remyelinisierung eines demyelinisierten Axons *in vivo*, die durch ein Verfahren erzeugt wurden, das die Schritte umfasst:
(a) Isolieren von neuronalen Stammzellen aus dem Gewebe eines Spenders,
(b) Proliferieren der isolierten neuronalen Stammzellen in einem ersten Kulturmedium, das einen Wachstumsfaktor enthält, um Vorläuferzellen zu erzeugen,
(c) Differenzieren der Vorläuferzellen in einem zweiten Kulturmedium, das im Wesentlichen frei von dem Wachstumsfaktor ist, um Oligodendrocyten zu erzeugen.

16. Oligodendrocyten für die Verwendung nach Anspruch 15, wobei das Kulturmedium aus Schritt (c) Serum enthält.

17. Oligodendrocyten für die Verwendung nach Anspruch 15, wobei der Wachstumsfaktor der Epidermis-Wachstumsfaktor ist.

18. Oligodendrocyten für die Verwendung nach Anspruch 15, wobei die Verwendung die Verabreichung der Oligodendrocyten an einen Patienten mit einer Demyelinisierungserkrankung umfasst.

19. Oligodendrocyten für die Verwendung nach Anspruch 18, wobei die Erkrankung ausgewählt ist aus der Gruppe bestehend aus Multipler Sklerose, disseminierter perivenöser Enzephalomyelitis, optischer Neuromyelitis, konzentrischer Sklerose, akuter disseminierter Enzephalomyelitis (*acute disseminated encaphalomyelitides*), Postenzephalomyelitis, akuter hämorrhagischer Leukoenzephalopathie, progressiver multifokaler Leukoenzephalopathie, Neuronitis, diphtherischer Neuropathie (*diphtheria neuropathy*), Pelizaeus-Merzbacher Erkrankung, optischer Neuromyelitis, diffuser Zerebralsklerose, zentraler pontiner Myelinolyse und Leukodystrophie.

20. Verwendung von Oligodendrocyten zur Herstellung einer Zellzusammensetzung zur Durchführung einer Remyelinisierung eines demyelinisierten Axons *in vivo,* die durch ein Verfahren erzeugt wurden, das die Schritte umfasst:
(a) Bereitstellen von neuronalen Stammzellen,
(b) Proliferieren der neuronalen Stammzellen in einem ersten Kulturmedium, das einen Wachstumsfaktor enthält, um Vorläuferzellen zu erzeugen,
(c) Differenzieren der Vorläuferzellen in einem zweiten Kulturmedium, das im Wesentlichen frei von dem Wachstumsfaktor ist, um Oligodendrocyten zu erzeugen.

21. Verwendung nach Anspruch 20, wobei das Kulturmedium aus Schritt (c) Serum enthält.

22. Verwendung nach Anspruch 20, wobei der Wachstumsfaktor der Epidermis-Wachstumsfaktor ist.

23. Verwendung nach Anspruch 20, wobei die Oligodendrocyten für die Verabreichung an einen Patienten mit einer Demyelinisierungserkrankung bestimmt sind.

24. Verwendung nach Anspruch 23, wobei die Demyelinisierungserkrankung ausgewählt ist aus der Gruppe bestehend aus Multipler Sklerose, disseminierter perivenöser Enzephalomyelitis, optischer Neuromyelitis, konzentrischer Sklerose, akuter disseminierter Enzephalomyelitis (*acute disseminated encaphalomyelitides*), Postenzephalomyelitis, akuter hämorrhagischer Leukoenzephalopathie, progressiver multifokaler Leukoenzephalopathie, Neuronitis, diphtherischer Neuropathie (*diphtheria neuropathy*), Pelizaeus-Merzbacher Erkrankung, optischer Neuromyelitis, diffuser Zerebralsklerose, zentraler pontiner Myelinolyse und Leukodystrophie.

25. Verfahren zur Herstellung von Gliazellen, umfassend die Schritte:
(a) Entnehmen von neuronalen Stammzellen von einem Spender,
(b) Proliferieren der isolierten neuronalen Stammzellen in einem ersten Kulturmedium, das einen Wachstumsfaktor enthält, um Vorläuferzellen zu erzeugen, und
(c) Differenzieren der Vorläuferzellen in einem zweiten Kulturmedium, das im Wesentlichen frei von dem Wachstumsfaktor ist, um Gliazellen zu erhalten.

26. Verfahren nach Anspruch 25, wobei das Kulturmedium aus Schritt (c) Serum enthält.

27. Verfahren nach Anspruch 25, wobei die Gliazellen Oligodendrocyten sind.

28. Verfahren nach Anspruch 25, wobei die Gliazellen Astrocyten sind.

29. Verfahren nach Anspruch 25, wobei der Wachstumsfaktor der Epidermis-Wachstumsfaktor ist.

30. Verfahren nach Anspruch 25, wobei die Vorläuferzellen aus Schritt (b) in Neurokörpern vorliegen.

## Revendications

1. Cellules souches neurales isolées du tissu d'un donneur, qui ont proliféré dans un milieu de culture contenant un facteur de croissance pour produire des cellules précurseurs, cellules qui sont recueillies afin d'être utilisées pour provoquer la remyélinisation d'un axone démyélinisé in vivo.

2. Cellules souches neurales destinées à être utilisées suivant la revendication 1, dans lesquelles le facteur de croissance est le facteur de croissance épidermique.

3. Cellules souches neurales destinées à être utilisées suivant la revendication 1, dans lesquelles les cellules précurseurs sont dans des neurosphères.

4. Cellules souches neurales destinées à être utilisées suivant la revendication 1, dans lesquelles les cellules précurseurs recueillies sont réadministrées au donneur pour provoquer la remyélinisation d'un axone démyélinisé.

5. Cellules souches neurales destinées à être utilisées suivant la revendication 1, dans lesquelles les cellules précurseurs recueillies sont administrées à un patient humain.

6. Cellules souches neurales destinées à être utilisées suivant la revendication 1 ou la revendication 2, dans lesquelles l'utilisation comprend l'administration des cellules précurseurs recueillies à un patient souffrant d'une maladie démyélinisante.

7. Cellules souches neurales destinées à être utilisées suivant la revendication 6, dans lesquelles la maladie démyélinisante est choisie dans le groupe consistant en la sclérose en plaques, l'encéphalomyélite périveineuse disséminée, la neuromyélite optique, la sclérose concentrique, l'encéphalomyélite aiguë disséminée, la post-encéphalomyélite, la leuco-encéphalopathie hémorragique aiguë, la leuco-encéphalopathie multifocale progressive, la polynévrite idiopathique, la neuropathie diphtérique, la maladie de Pelizaeus-Merzbacher, la neuromyélite optique, la sclérose cérébrale diffuse, la myélinolyse centropontine et la leucodystrophie.

8. Utilisation de cellules souches neurales isolées qui ont été amenées à proliférer dans un milieu de culture contenant un facteur de croissance pour produire des cellules précurseurs, cellules qui sont recueillies, dans la production d'une préparation de cellules pour provoquer la remyélinisation d'un axone démyélinisé in vivo.

9. Utilisation suivant la revendication 8, dans laquelle le facteur de croissance est le facteur de croissance épidermique.

10. Utilisation suivant la revendication 8, dans laquelle les cellules précurseurs sont dans des neurosphères.

11. Utilisation suivant la revendication 8, dans laquelle les cellules souches neurales ont été isolées du tissu d'un donneur et lesdites cellules précurseurs recueilies sont destinées à l'administration à ce donneur.

12. Utilisation suivant la revendication 8, dans laquelle les cellules précurseurs recueillies sont destinées à l'administration à un patient humain.

13. Utilisation suivant la revendication 8 ou la revendication 9, dans laquelle les cellules précurseurs recueillies sont destinées à l'administration à un patient souffrant d'une maladie démyélinisante.

14. Utilisation suivant la revendication 13, dans laquelle la maladie est choisie dans le groupe consistant en la sclérose en plaques, l'encéphalomyélite périveineuse disséminée, la neuromyélite optique, la sclérose concentrique, l'encéphalomyélite disséminée aiguë, la post-encéphalomyélite, la leuco-encéphalopathie hémorragique aiguë, la leuco-encéphalopathie multifocale progressive, la polynévrite iodopathique, la neuropathie diphtérique, la malade de Pelizaeus-Merzbacher, la neuromyélite optique, la sclérose cérébrale diffuse, la myélinolyse centropontine et la leucodystrophie.

15. Oligodendrocytes qui ont été produits par un procédé comprenant les étapes consistant :
(a) à isoler des cellules souches neurales du tissu d'un donneur ;
(b) à faire proliférer les cellules souches neurales isolées dans un premier milieu de culture contenant un facteur de croissance pour la production de cellules précurseurs ;
(c) à provoquer la différenciation des cellules précurseurs dans un second milieu de culture qui est pratiquement dépourvu dudit facteur de croissance afin de produire les oligodendrocytes, destinés à être utilisés pour provoquer la remyélinisation d'un axone démyélinisé in vivo.

16. Oligodendrocytes destinés à être utilisés suivant la revendication 15, dans lesquels le milieu de culture de l'étape (c) contient du sérum.

17. Oligodendrocytes destinés à être utilisés suivant la revendication 15, dans lesquels le facteur de croissance est le facteur de croissance épidermique.

18. Oligodendrocytes destinés à être utilisés suivant la revendication 15, dans lesquels l'utilisation comprend l'administration desdits oligodendrocytes à un patient souffrant d'une maladie démyélinisante.

19. Oligodendrocytes destinés à être utilisés suivant la revendication 18, dans lesquels la maladie est choisie dans le groupe consistant en la sclérose en plaques, l'encéphalomyélite périveineuse disséminée, la neuromyélite optique, la sclérose concentrique, l'encéphalomyélite disséminée aiguë, la post-encéphalomyélite, la leuco-encéphalopathie hémorragique aiguë, la leuco-encéphalopathie multifocale progressive, la polynévrite idiopathique, la neuropathie diphtérique, la maladie de Pelizaeus-Merzbacher, la neuromyélite optique, la sclérose cérébrale diffuse, la myélinolyse centropontine et la leucodystrophie.

20. Utilisation d'oligodendrocytes qui ont été produits par un procédé comprenant les étapes consistant :
(a) à obtenir des cellules souches neurales ;
(b) à faire proliférer les cellules souches neurales dans un premier milieu de culture contenant un facteur de croissance pour produire des cellules précurseurs ;
(c) à provoquer la différenciation des cellules précurseurs dans un second milieu de culture qui est pratiquement dépourvu dudit facteur de croissance afin de produire des oligodendrocytes, dans la production d'une préparation de cellules destinée à provoquer la remyélinisation d'un axone démyélinisé in vivo.

21. Utilisation suivant la revendication 20, dans laquelle le milieu de culture de l'étape (c) contient du sérum.

22. Utilisation suivant la revendication 20, dans laquelle le facteur de croissance est le facteur de croissance épidermique.

23. Utilisation suivant la revendication 20, dans laquelle les oligodendrocytes sont destinés à l'administration à un patient souffrant d'une maladie démyélinisante.

24. Utilisation suivant la revendication 23, dans laquelle la maladie démyélinisante est choisie dans le groupe consistant en la sclérose en plaques, l'encéphalomyélite périveineuse disséminée, la neuromyélite optique, la sclérose concentrique, l'encéphalomyélite disséminée aiguë, la post-encéphalomyélite, la leuco-encéphalopathie hémorragique aiguë, la leuco-encéphalopathie multifocale progressive, la polynévrite idiopathique, la neuropathie diphtérique, la maladie de Pelizaeus-Merzbacher, la neuromyélite optique, la sclérose cérébrale diffuse, la myélinolyse centropontine et la leucodystrophie.

25. Procédé pour la production de cellules gliales, comprenant les étapes consistant :
(a) à prendre des cellules souches neurales d'un donneur,
(b) à faire proliférer les cellules souches neurales isolées dans un premier milieu de culture contenant un facteur de croissance pour produire des cellules précurseurs, et
(c) à provoquer la différenciation des cellules précurseurs dans un second milieu de culture qui est pratiquement dépourvu dudit facteur de croissance afin d'obtenir des cellules gliales.

26. Procédé suivant la revendication 25, dans lequel le milieu de culture dans l'étape (c) contient du sérum.

27. Procédé suivant la revendication 25, dans lequel les cellules gliales sont des oligodendrocytes.

28. Procédé suivant la revendication 25, dans lequel les cellules gliales sont des astrocytes.

29. Procédé suivant la revendication 25, dans lequel le facteur de croissance est le facteur de croissance épidermique.

30. Procédé suivant la revendication 25, dans lequel les cellules précurseurs de l'étape (b) sont dans des neurosphères.
